(19) Europäisches Patentamt
European Patent Office
Office européen des brevets

(11) Veröffentlichungsnummer : **0 254 268 B2**

(12) **NEUE EUROPÄISCHE PATENTSCHRIFT**

(45) Veröffentlichungstag der neuen Patentschrift : **12.01.94 Patentblatt 94/02**

(51) Int. Cl.$^5$ : **C07H 19/073**, C07H 19/173, A61K 31/70

(21) Anmeldenummer : **87110542.5**

(22) Anmeldetag : **21.07.87**

# (54) Fluorierte Nucleoside, ihre Herstellung und ihre pharmazeutische Verwendung gegen AIDS.

(30) Priorität : **24.07.86 DD 292826**
**08.05.87 DD 302573**
**03.06.87 DD 303489**

(43) Veröffentlichungstag der Anmeldung : **27.01.88 Patentblatt 88/04**

(45) Bekanntmachung des Hinweises auf die Patenterteilung : **19.09.90 Patentblatt 90/38**

(45) Bekanntmachung des Hinweises auf die Entscheidung über den Einspruch : **12.01.94 Patentblatt 94/02**

(84) Benannte Vertragsstaaten : **BE CH DE ES FR GB GR IT LI LU NL SE**

(56) Entgegenhaltungen :
**WO-A-88/00050**
**DD-A- 158 903**
**DD-A- 209 197**
**GB-A- 1 189 973**
**TETRAHEDRON, Band 27, 1971, Seiten 2463-2472, Pergamon Press, Oxford, GB; G. ETZOLD et al.: "Nucleoside von Fluorzuckern-VI Synthese und Reaktivität von 3'-Fluor-und 3'-Chlor-3'-Desoxy-Thymidin"**
**JOURNAL OF MEDICINAL CHEMISTRY, Band 15, Nr. 10, 1972, Seiten 1092-1093, Columbus, Ohio, US; K. MIYAI et al.: "Synthesis of 9-(3-deoxy-3fluoro-beta-D-arabinofuranosyl)adenine"**
**CHEMICAL ABSTRACTS, Band 93, 1980, Seite 24, Zusammenfassung Nr. 160970q, Columbus, Ohio, US; R.F. BRUNS: "Adenosine receptor activation in human fibroblasts: nucleoside agonists and atagonists", & CAN. J. PHIYSIOL. PHARMACOL. 1980, 58(6), 673-91**

(56) Entgegenhaltungen :
**CHEMICAL ABSTRACTS, Band 69, 1968, Seite 1026, Zusammenfassung Nr. 10663g, Columbus, Ohio, US; J.A. WRIGHT et al.: "Fluorocarbohydrates. XVIII. 9-(3-deoxy-3-fluoro-beta-D-xylofuranosyl)adenine and 9-(3-deoxy-3-fluoro-alpha-D-arabinofuranosyl)adenine", & CARBOHYD. RES. 1968, 6(3), 347-54**
**J.BIOL.CHEM., 265/2, 1987, pp. 2187-2189**

(73) Patentinhaber : **THE WELLCOME FOUNDATION LIMITED**
**Unicorn House 160 Euston Road**
**London NW1 2BP (GB)**

(72) Erfinder : **Matthes, Eckart, Dr.**
**Karower Chaussee 129**
**DD-1115 Berlin (DD)**
Erfinder : **Lehmann, Christine**
**Walter-Friedrich-Strasse 5**
**DD-1115 Berlin (DD)**
Erfinder : **Scholz, Dieter, Dr.**
**Heinrich-Roller-Strasse 16**
**DD-1055 Berlin (DD)**
Erfinder : **von Janta-Lipinski, Dr.**
**Pradelstrasse 6**
**DD-1100 Berlin (DD)**
Erfinder : **Gaertner, Klaus**
**Karower Chaussee 157**
**DD-1115 Berlin (DD)**
Erfinder : **Langen, Peter, Prof. Dr.**
**Karower Chaussee 219**
**DD-1115 Berlin (DD)**
Erfinder : **Rosenthal, Hans-Alfred, Prof. Dr.**
**Märkisches Ufer 14**
**DD-1020 Berlin (DD)**

(74) Vertreter : **TER MEER - MÜLLER - STEINMEISTER & PARTNER**
**Mauerkircherstrasse 45**
**D-81679 München (DE)**



Jouve, 18, rue Saint-Denis, 75001 PARIS

## Beschreibung

Die Erfindung betrifft 3'-fluorierte Pyrimidin- und Purinnucleoside, die gegen HIV-Infektionen beim Menschen (AIDS) wirksam sind, ihre Herstellung und ihre Verwendung in Form entsprechender pharmazeutischer Mittel.

AIDS ist eine erst seit wenigen Jahren bekannte, durch das HIV (HTLV III/LAV; Human T-Lymphotropic Virus Type III/Lymphadenopathy-Associated Virus) verursachte und zum Tode führende Infektionskrankheit. Als ursächlich muß die Zerstörung der T-Helfer-Zellen durch das AIDS-Virus angesehen werden. Als Folge der daraus resultierenden Abwehrschwäche treten schwere opportunistische Infektionen, das Kaposi-Sarkom und eine sog. AIDS-Encephalopathie auf. Eine wirksame und verträgliche antivirale Therapie fehlt bisher. Angriffspunkt dafür kann u.a. die viruscodierte Reverse Transcriptase sein, ein Enzym, dessen Hemmung die weitere intracelluläre Virusvermehrung verhindern und damit seine Ausbreitung im Körper stoppen könnte. Die ersten klinisch erprobten Hemmstoffe der HIV-Revertase, wie z.B. Suramin (Germanin®) und HPA 23, haben die notwendige Verträglichkeit und die erhoffte Wirksamkeit noch nicht erreicht, lediglich das 3'-Azidothymidin ($N_3$TdR) (DE-A 3 608 606) besitzt bei AIDS-Patienten mit einer Pneumocystis-carinii-Pneumonie eindeutig lebensverlängernde Wirkungen, die von Verbesserungen klinischer und neurologischer Befunde sowie der zeitweisen Wiederherstellung bestimmter immunologischer Funktionen begleitet sind (Mitsuya et al., Nature 325 (1987) 773). Demgegenüber machen toxische Nebenwirkungen des 3'-Azidothymidins auf das Knochenmark bei etwa 50% der behandelten Patienten Bluttransfusionen erforderlich, was besonders das Bedürfnis nach selektiveren und zugleich wirksameren Hemmstoffen der HIV-Reverse Transcriptase erweist.

Es waren ferner bereits einige Purin- und Pyrimidinderivate per se bekannt, ohne daß allerdings ihre Verwendbarkeit zur AIDS-Bekämpfung erkannt worden war, nämlich 2',3'-Didesoxy-3'-fluorthymidin (3'-FTdR) (G. Etzold, R. Hintsche, G. Kowollik und P. Langen, Tetrahedron (London) 27 (1971) 2463), 2',3'-Didesoxy-3'-fluorcytidin (G. Kowollik, G. Etzold, M. v. Janta-Lipinski, K. Gaertner und P. Langen, J. prakt. Chemie 315 (1973) 895), 3'-Desoxy-3'-fluorarabinosylcytosin (G. Kowollik, Habilitationsschrift, Berlin 1976) und 3'-Desoxy-3'-fluorarabinosyladenin (K. Miyai, R.K. Robbins und R.L. Tolman, J. med. Chemistry 15 (1972) 1962). Gleiches gilt für die aus GB-B 1 189 973 bekannten 2',3'-Didesoxy-3'-fluorpyrimidinnucleoside der Formel

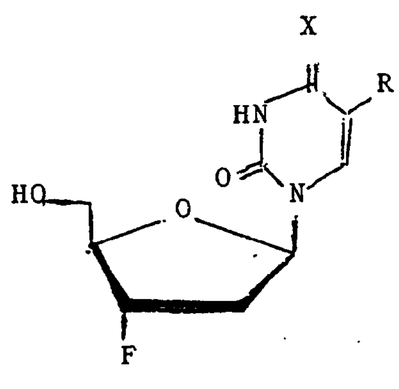

in der X Sauerstoff, Schwefel oder Imino und R H, OH, SH, Halogen oder Alkyl bedeuten.

Der Erfindung liegt die Aufgabe zugrunde, neuartige Wirkstoffe, ihre Herstellung und entsprechende pharmazeutische Mittel bzw. die Verwendung derartiger Wirkstoffe zur Prophylaxe und therapeutischen Behandlung von AIDS bzw. HIV-Infektionen, insbesondere in der Humanmedizin, anzugeben.

Die Aufgabe wird gemäß den Ansprüchen 1, 8, 9 und 10 gelöst. Vorteilhafte Weiterbildungen sind Gegenstand der abhängigen Ansprüche.

Die erfindungsgemäßen 3'-fluorierten Pyrimidin- bzw. Purinnucleoside besitzen die allgemeine Formel I,

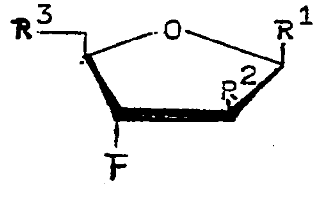

(I),

worin bedeuten:

$R^1$ einen N-1-glykosidisch gebundenen Rest, der von Thymin, Uracil, einem 5-substituierten Uracil, Cytosin oder einem 5-substituierten Cytosinderivat abgeleitet ist,
oder einen N-9-glykosidisch gebundenen Rest, der von 2-Fluoradenin, 2,6-Diaminopurin, 2-Aminopurin, 6-Thioguanin oder 7-Deazaadenin abgeleitet ist,
$R^2$ H oder OH und
$R^3$ OH, O-Acetyl, O-Palmitoyl, Triphosphat als freie Säure oder in Form entsprechender Alkali-, Ammonium- bzw. Alkylammoniumsalze oder eine Prekursor-Gruppe für die Hydroxygruppe,
wobei diejenigen Verbindungen der Formel I ausgenommen sind, in der
$R^1$ einen N-1-glykosidisch gebundenen Rest, der von Thymin, Uracil, einem 5-substituierten Uracil, Cytosin oder einem 5-substituierten Cytosinderivat abgeleitet ist,
$R^2$ H und
$R^3$ OH, O-Acetyl, O-Palmitoyl, Triphosphat in Form entsprechender Alkali-, Ammonium- bzw. Alkylammoniumsalze oder eine Prekursor-Gruppe für die Hydroxygruppe.
bedeuten.

Diese Verbindungen der Formel I stellen Wirkstoffe gegen HIV-Infektionen dar und können als solche sowie in Form pharmazeutischer Mittel zusammen mit pharmazeutisch geeigneten Hilfs- und/oder Trägerstoffen zur Prophylaxe und Therapie von AIDS, besonders in der Humanmedizin, vorteilhaft eingesetzt werden.

Sie weisen gegenüber dem Stand der Technik geringere Toxizität bzw. Nebenwirkungen auf.

Die erfindungsgemäßen Verfahren zur Herstellung der 3'-fluorierten Pyrimidinnucleoside der Formel I sind dadurch gekennzeichnet, daß man

- zur Herstellung von ggfs. 5-substituierten 2',3'-Didesoxy-3'-fluoruridinen entsprechende, ggfs. 5-substituierte 2',3'-Anhydro-2'-desoxyuridine mit HF umsetzt;
- zur Herstellung von 5-substituierten 2',3'-Didesoxy-3'-fluoruridinen oder
  2',3'-Didesoxy-3'-fluorthymidin (3'-FTdR) radikalisch bromiert und das erhaltene 5-Brommethyl-2',3'-didesoxy-3'-fluoruridin entsprechend nucleophil substituiert;
- zur Herstellung von ggfs. 5-substituierten 2',3'-Didesoxy-3'-fluorcytidinen entsprechende, ggfs. 5-substituierte Uracilderivate bzw. Uridine in die Triazolylderivate überführt und daraus die Zielverbindungen herstellt;
- zur Herstellung von ggfs. 5-substituierten 3'-Desoxy-3'-fluoroarabinosylcytosinen die entsprechenden, ggfs. 5-substituierten Cytosinderivate in die Triazolylderivate überführt und daraus die Zielverbindungen herstellt und
- zur Herstellung von 2- bzw. 2,6-substituierten 2',3'-Didesoxy-3'-fluoropurinribosiden die entsprechenden Xyloribofuranosylderivate, die in 5'-Position acyliert sind, mit Dialkylaminoschwefelfluorid umsetzt oder
  den fluorierten Zuckerrest aus 2',3'-Didesoxy-3'-fluorthymidin (3'-FTdR) durch Transglykosidierung auf das entsprechend modifizierte Purinderivat überträgt.

Erfindungsgemäße Verbindungen der Formel I mit besonders günstigen Eigenschaften sind 2',3'-Didesoxy-3'-fluor-5-bromuridin, 2',3'-Didesoxy-3'-fluor-5-ethyluridin, 2',3'-Didesoxy-3'-fluor-2-fluoradenosin, 2',3'-Didesoxy-3'-fluor-6-thioguanosin, 2',3'-Didesoxy-3'-fluor-2,6-diaminopurinribosid und 2',3'-Didesoxy-3'-fluor-2-aminopurinribosid.

Eine Behandlung oder Prophylaxe von AIDS in der Humanmedizin besteht gemäß der Erfindung in der Verabreichung einer wirksamen Menge einer oder mehrerer Verbindungen der Formel I als solche oder in Form geeigneter galenischer Formulierungen.

Die erfindungsgemäßen pharmazeutischen Mittel, die insbesondere zur Prophylaxe und Therapie von AIDS in der Humanmedizin geeignet sind, enthalten entsprechend mindestens ein 3'-fluoriertes Pyrimidin- bzw. Purinnucleosid der Formel I wie oben definiert als Wirkstoff neben einem oder mehreren pharmazeutisch geeigneten Hilfs- und/oder Trägerstoffen und gegebenenfalls anderen therapeutischen Mitteln oder Wirkstoffen.

Die Erfindung lehrt ferner die Verwendung von 3'-fluorierten Pyrimidin- bzw. Purinnucleosiden der Formel I, in der
$R^1$ einen N-1-glykosidisch gebundenen, von Thymin, Uracil, einem 5-substituierten Uracil, Cytosin oder einem 5-substituierten Cytosinderivat abgeleiteten Rest,
$R^2$ H und
$R^3$ OH
bedeuten, sowie von 2',3'-Didesoxy-3'-fluorthymidin (3'-FTdR), 2',3'-Didesoxy-3'-fluorcytidin, 3'-Desoxy-3'-fluorarabinosylcytosin
und 3'-Desoxy-3'-fluorarabinosyladenin zur Herstellung pharmazeutischer Mittel zur Prophylaxe und zur Bekämpfung der erworbenen Immunschwäche AIDS, insbesondere in der Humanmedizin.

Die Mittel werden in Form von Dosiereinheiten oder Vielfachen davon hergestellt. Die eingesetzten Hilfs- und Trägerstoffe müssen verträglich, mit den anderen Bestandteilen der Mittel kompatibel und für den Patienten unschädlich sein.

Zu den erfindungsgemäßen galenischen Formulierungen bzw. Applikationsformen der pharmazeutischen Mittel gehören solche, die für die orale, rektale, nasale, topische, vaginale oder parenterale (einschließlich subkutaner, intramuskulärer, intravenöser und intradermaler) Verabreichung geeignet sind. Ein bzw. mehrere Wirkstoffe werden mit dem Träger, der aus mehreren Begleitbestandteilen zusammengesetzt sein kann, in Kontakt gebracht und, falls erforderlich, in eine entsprechende galenische Form übergeführt.

Die pharmazeutischen Mittel für orale Verabreichung werden in Form von Dragées, Tabletten, Kapseln, als Pulver oder als Granulate hergestellt, die jeweils eine bestimmte Menge des Wirkstoffs enthalten. Ebenso können sie als Lösungen oder als Suspensionen formuliert werden. Gegebenenfalls werden Geschmacksmittel und/oder andere übliche Additive zugesetzt.

Mittel für die rektale Verabreichung werden als Zäpfchen mit einer geeigneten Grundlage hergestellt. Arzneimittel für eine vaginale Verabreichung werden z.B. als Pessare, Tampons, Cremen, Gele, Pasten, Schäume oder Spray-Produkte formuliert.

Die Mittel für die parenterale Verabreichung können eine Dosiereinheit des Wirkstoffs oder das Mehrfache davon enthalten und beispielsweise in Ampullen, Phiolen oder in einem gefriergetrockneten Zustand lagerfähig gemacht sein. Unmittelbar vor Gebrauch hergestellte Injektionslösungen und Suspensionen können aus sterilen Pulvern, Granulaten und Tabletten erhalten werden, z.B. durch Lösen der Substanz in physiologischer Kochsalzlösung, Glucose oder anderen für die i.v.-Injektion bzw. Infusion geeigneten Medien.

Zur Behandlung von AIDS-Patienten wird beispielsweise aus 3'-FTdR und physiologischer Kochsalzlösung die erforderliche Menge einer 0,3-%igen Lösung hergestellt, die sterilisiert und dem Patienten innerhalb einer Stunde intravenös infundiert wird. Die Infusion wird über mindestens 8 bis 10 Tage alle 4 bis 8 Stunden wiederholt.

Erfindungsgemäß besonders geeignete Wirkstoffe sind 2',3'-Didesoxy-3'-fluorthymidin (3'-FTdR), 2',3'-Didesoxy-3'-fluor-5-bromuridin, 2',3'-Didesoxy-3'-fluorcytidin, 2',3'-Didesoxy-3'-fluor-5-ethyluridin, 2',3'-Didesoxy-3'-fluor-2-fluoradenosin, 2',3'-Didesoxy-3'-fluor-6-thioguanosin, 2',3'-Didesoxy-3'-fluor-2,6-diaminopurinribosid, 2',3'-Didesoxy-3'-fluor-2-aminopurinribosid, 3'-Didesoxy-3'-fluorarabinosylcytosin und 3'-Desoxy-3'-fluorarabinosyladenin.

Als besonders aktiv erwies sich davon das 2',3'-Didesoxy-3'-fluorthymidin (3'-FTdR). Seine Wirksamkeit wird wie folgt belegt:

## 1. Hemmung der HIV-assoziierten Reverse Transcriptase

Zunächst wurde das Triphosphat (3'-FdTTP) des 3'-FTdR auf seine Fähigkeit untersucht, die Polymerisation eines PolyA-oligodT-Templats durch die HIV-assoziierte Reverse Transcriptase zu hemmen. Dabei wurde gefunden (Fig. 1), daß die Konzentration, die für eine 50-%ige Hemmung dieses viralen Enzyms erforderlich ist ($ID_{50}$), bei 0,05 $\mu$M ($\mu$mol/l) liegt, und daß eine praktisch vollständige Hemmung bei 0,9 bis 1,0 $\mu$M erreicht wird. Damit gehört 3'-FdTTP zu den stärksten bisher gefundenen Hemmstoffen der HIV-Reverse-Transcriptase.

Untersuchungen zum Wirkungsmechanismus zeigten, daß 3'-FdTTP als kompetitiver Hemmstoff die Bindungsstelle des Substrats besetzt, während sein Einbau in den Primer und ein damit verbundener Kettenabbruch, wenn überhaupt, bei der Hemmung nur eine untergeordnete Rolle spielt.

Im Gegensatz zu 3'-FdTTP erwies sich 2',3'-Didesoxy-3'-chlorthymidintriphosphat gegenüber der HIV-assoziierten Reverse Transcriptase im gleichen Konzentrationsbereich als völlig unwirksam, was darauf hinweist, daß der 3'-Substituent dieser Nucleotidanaloga sehr spezifische Funktionen am Enzym zu erfüllen hat und nicht beliebig austauschbar ist, wenn eine hohe Wirksamkeit erhalten bleiben soll.

Zugleich wird daraus ersichtlich, daß für die Wirksamkeit der erfindungsgemäßen Verbindungen der Formel I sowie der erfindungsgemäß zur AIDS-Prophylaxe und -Therapie verwendeten bekannten Nucleoside keine Erwartungshaltung bestand, zumal über die Bedeutung der Art des 3'-Substituenten keinerlei Informationen vorlagen.

## 2. Wirkung von 3'-FdTTP auf die cellulären DNA-Polymerasen $\alpha$ und $\beta$

Die starke Hemmwirkung von 3'-FdTTP gegenüber der HIV-Reverse-Transcriptase läßt sich therapeutisch nur ausnutzen, wenn die cellulären DNA-Polymerasen, insbesondere die für die Replikation der cellulären DNA zuständige DNA-Polymerase-$\alpha$, weitgehend unbeeinflußt bleiben. Die Untersuchungen zeigen, daß dies für die DNA-Polymerase-$\alpha$ zutrifft (Fig. 2). Eine 50-%ige Hemmung dieses cellulären Enzyms wird auch bei 200

µM 3'-FdTTP noch nicht erreicht, was bedeutet, daß die DNA-Polymerase-α mehr als 4000-mal weniger empfindlich ist als die HIV-Reverse-Transcriptase.

Für die DNA-Polymerase-β, die für die celluläre DNA-Reparatur verantwortlich gemacht wird, beträgt die $ID_{50}$ etwa 2,2 µM; für eine 50-%ige Hemmung dieses Enzyms ist also eine 44-mal höhere Konzentration erforderlich als für eine gleich starke Hemmung der HIV-Reverse-Transcriptase.

3. Intracelluläre Phosphorylierung von 3'-FTdR

Entscheidend für die Wirksamkeit von 3'-FTdR ist die Fähigkeit der infizierten Zellen, diese nur in Nucleosidform anwendbare Substanz zum entsprechenden Triphosphat zu phosphorylieren. Eine mangelnde intracelluläre Phosphorylierung von 3'-modifizierten Desoxynucleosiden kann daher durchaus, wie im Falle des 2',3'-Didesoxythymidins, die Wirksamkeit einer im HIV-Revertase-Test hoch effektiven Verbindung stark herabsetzen. Die Phosphorylierung von 3'-FTdR wurde an Zellen verschiedener Species untersucht. Wie die nachstehende Tabelle 1 zeigt, wird 1 µM FTdR von den untersuchten Zellen während einer 4-stündigen Inkubation mit sehr unterschiedlicher Effektivität zum Triphosphat umgesetzt (FTMP, FTDP und FTTP entsprechendes Mono-, Di- bzw. Triphosphat).

Tabelle 1

Bildung von Nucleotiden aus 1 µM $^3H$-3'-FTdR in Zellen verschiedener Species nach 4-stündiger Inkubation

| | | (pmol/$10^6$ Zellen) | | |
|---|---|---|---|---|
| | Zellinie | FTMP | FTDP | FTTP |
| Mensch | MT-4 | 2,9 | 0,2 | 1,3 |
| | REH | 3,5 | 0,35 | 2,5 |
| Ratte | Zajdela-Hepatom | 3,0 | 3,6 | 8,8 |
| Maus | EMAC* | 1,2 | 4,4 | 19,2 |

* Ehrlich-Mäuse-Ascites-Tumor.

Obwohl für einen quantitativen Vergleich auch unterschiedliche Zellzykluszeiten zu berücksichtigen sind und nur der während der 4-stündigen Inkubation in der $G_1$- bzw. S-Phase des Zellzyklus befindliche Anteil der Zellen zur Phosphorylierung in der Lage ist, sind die für die menschlichen Zellinien MT-4 und REH gefundenen Triphosphat-Mengen von 1 bis 2 pmol/$10^6$ Zellen als völlig ausreichend anzusehen. Auch für nichtinfizierte bzw. HTLV-$III_B$-infizierte H9-Zellen und LAV-II-infizierte CEM-Zellen liegen die ermittelten 3'-dFTTP-Konzentrationen in diesem Bereich, so daß eine AIDS-Virus-Infektion von T-Zellen die Phosphorylierbarkeit von 3'-FTdR offenbar nicht verändert. Im Vergleich dazu wird die Phosphorylierung einer 50 µM $N_3$-TdR-Lösung zum Triphosphat durch HTLV-$III_B$-infizierte H9-Zellen während einer 24-stündigen Inkubation mit nur 0,9 pmol/$10^6$ Zellen angegeben (Furman et al., Proc. Natl. Acad. Sci. USA 83 (1986) 8333). Gleichzeitig kommt es zu einem erheblichen Anstau von $N_3$-TdR-monophosphat (460 pmol/$10^6$ Zellen), was auf eine starke Hemmung der Thymidylat-Kinase hinweist und erhebliche, die celluläre DNA-Synthese beeinträchtigende Veränderungen des Desoxynucleosidtriphosphat-Substratpools nach sich zieht (Furman et al., loc. cit.). 3'-FTdR fehlt dieser gravierende Effekt auf die Thymidylat-Kinase (vgl. Tab. 1), so daß die starken cellulären Pool-Veränderungen nicht zu erwarten sind, obwohl 3'-FTdR gegenüber $N_3$TdR eindeutig besser phosphoryliert wird.

4. Beständigkeit von 3'-FTdR gegenüber phosphorolytischer Spaltung

Einige Thymidin-Antimetabolite, wie Joddesoxyuridin, Bromdesoxyuridin oder Bromvinyldesoxyuridin, werden von der menschlichen Thymidin-Phosphorylase in so großem Umfang zu unwirksamen Pyrimidinbasen und Zuckerphosphaten gespalten, daß ihre systemische Anwendung problematisch werden kann. 3'-FTdR wurde daher auf seine Spaltbarkeit durch die Thymidin-Phosphorylase aus der menschlichen Milz untersucht.

Dabei ergab sich, daß eine 1 µM 3'-FTdR-Lösung innerhalb von 3 h nur zu etwa 3 bis 5% gespalten wird und damit 3'-FTdR im Vergleich zu Thymidin (90% Spaltung in 2 h) als gegenüber der Wirkung dieses Enzyms nahezu resistent anzusehen ist.

5. Hemmung des cytopathischen Effekts von HIV auf MT-4-Zellen

Um die tatsächliche celluläre Wirksamkeit von 3'-FTdR zu ermitteln, wurde untersucht, inwieweit 3'-FTdR den cytopathischen Effekt von HIV aufheben kann. Dazu wurden ca. 40 000 MT-4-Zellen (Harada et al., Science 229 (1985) 563) in 200 µl RPMI-Medium und 10% Kälberfetalserum mit HIV infiziert (Titer: 0,013 mol) und nach 5-tägiger Inkubation anhand der Trypanblaufärbbarkeit die lebenden von den toten Zellen unterschieden und gezählt. Die Konzentration, die für eine 50%-ige Hemmung des zelltötenden Effekts des Virus erforderlich ist ($ED_{50}$), beträgt 3,8 nM für 3'-FTdR und im direkten Vergleich 6,4 nM für $N_3$-TdR. Für 2',3'-Didehydro-2',3'-didesoxythymidin ist eine $ED_{50}$ von 10 nM und für 2',3'-Didesoxycytidin eine $ED_{50}$ von 60 nM angegeben worden (M. Baba et al., Biochem. Biophys. Res. Commun. 142 (1987) 128). Damit ist 3'-FTdR bei den genannten MT-4-Zellen als derzeit wirksamste Verbindung anzusehen.

6. Cytotoxizität von 3'-FTdR in menschlichen Zellkulturen

3'-FTdR wurde ursprünglich als Cytostatikum entwickelt (P. Langen et al., Acta biol. med. germ. 23 (1969) 759) und ist daher in umfangreichen Studien auf cytostatische Wirkungen hin geprüft worden. Untersuchungen im Rahmen der vorliegenden Erfindung sowie Tests im NCI-Programm der USA (gegen 9 verschiedene Tiertumoren) haben für 3'-FTdR jedoch nur schwache cytostatische Wirkungen ergeben. Eine Ausnahme liegt bei Ehrlich-Mäuse-Ascites-Tumorzellen (EMAC-Zellen) vor, was möglicherweise mit ihrem hohen Phosphorylierungsvermögen für 3'-FTdR zusammenhängt (10-fach höher als bei menschlichen Zellen). Dabei ist die schnelle Reversibilität der cytostatischen Wirkungen an EMAC-Zellen als Besonderheit dieser Verbindung registriert worden (P. Langen et al., Europ. J. Cancer 14 (1978) 349).

Unter dem Aspekt einer möglichen Anwendung dieser Verbindung am Menschen wurden die antiproliferativen Wirkung von 3'-FTdR und im Vergleich dazu von $N_3$-TdR an menschlichen Zellinien getestet.

Tabelle 2 zeigt anhand der $ID_{50}$-Werte, daß der Einfluß sowohl von 3'-FTdR als auch von $N_3$-TdR auf die Proliferation verschiedener Zellinien sehr unterschiedlich ist.

Tabelle 2

Hemmung der Proliferation menschlicher Zellinien durch FTdR und $N_3$TdR

| | ($ID_{50}$, µM) | |
|---|---|---|
| Zellinie | FTdR | $N_3$-TdR |
| K-562 | 45 | 50 |
| REH | 150 | 150 [a] |
| K-37 | 100 [b] | 100 [c] |
| MT-4 | 1,8 | 3,5 [d] |

a) 30 % Hemmung bei 150 µM
b) 35 % Hemmung bei 100 µM
c) 23 % Hemmung bei 100 µM
d) Angabe von M. Baba et al., Biochem. Biophys. Res. Commun. 142 (1987) 128.

Vergleicht man 3'-FTdR mit $N_3$-TdR, so sind die Effekte sehr ähnlich, jedoch für $N_3$-TdR in jedem Fall etwas geringer als bei FTdR.

Die Vorteile von 3'-FTdR lassen sich wie folgt zusammenfassen:

1. Doppelte Wirksamkeit gegenüber $N_3$-TdR im cellulären Test.

2. Bessere Phosphorylierung durch menschliche T-Zellinien als bei $N_3$-TdR.
3. Resistenz gegenüber TdR-Phosphorylase.
4. Geringere Effekte auf TMP-Kinase und damit auf die dNTP-Substratpools als $N_3$-TdR.
5. Vertretbare und insbesondere reversible celluläre Toxizität.

Die Erfindung wird im folgenden anhand von Beispielen näher erläutert.

Beispiel 1

Herstellung von 5-substituierten 2',3'-Didesoxy-3'-fluoruridinen durch Spaltung der 2',3'-Anhydrobindung entsprechender Derivate

1-(2,3-Didesoxy-3-fluor-β-D-ribofuranosyl)-5-ethyluracil

Ein Gemisch von 2,38 g (10 mmol) 2',3'-Anhydro-1-(2-desoxy-5-O-acetyl-β-D-xylofuranosyl)-5-ethyluracil, 2,5 g Aluminiumtrifluorid und 300 ml 1,4-Dioxan, das 0,5% Fluorwasserstoff enthielt, wurde in einem Stahlgefäß 1,5 h auf 110°C erhitzt. Nach dem Abkühlen wurden 100 ml Wasser und 20 g $CaCO_3$ zur Reaktionslösung gegeben.

Die filtrierte Lösung wurde zu einem Sirup eingeengt. Dieser Sirup wurde in 50 ml Methanol, das bei 0°C mit Ammoniak gesättigt worden war, gelöst und 24 h bei Raumtemperatur belassen. Nach dem Verdampfen des Lösungsmittels im Vakuum wurde das erhaltene Öl einer Säulenchromatographie an Kieselgel unterzogen, wobei mit Chloroform/Methanol (Volumverhältnis 9:1) eluiert wurde. Nach dem Verdampfen des Lösungsmittels konnte die Titelverbindung aus den entsprechenden Fraktionen als Feststoff erhalten werden. F. 183-184°C; Massenspektrum: m/z 258 ($M^+$).

Beispiel 2

Herstellung 5-substituierter 2',3'-Didesoxy-3'-fluoruridine durch radikalische Bromierung der 5-Methylgruppe in 3'-FTdR und nachfolgende nucleophile Substitution des Broms

a) 5-(Brommethyl)-1-(5-O-acetyl-3-desoxy-3-fluor-β-D-ribofuranosyl)-uracil

5'-O-Acetyl-3'-fluorthymidin (2,88 g, 10 mmol) wurde in 250 ml 1,2-Dichlorethan bis zum vollständigen Auflösen unter Rückfluß erhitzt. Danach wurden mit einem Stickstoffatom 12 mmol elementares Brom in die Lösung geleitet, die mit einer Photolampe (500 W) bestrahlt wurde. Nach 2-3 h war die Reaktion beendet. Das Lösungsmittel wurde im Vakuum abgedampft, wobei ein viskoses Öl erhalten wurde. Dieses Öl enthielt die Titelverbindung in der für die folgenden Reaktionen ausreichender Reinheit.

b) 1-(2,3-Didesoxy-3-fluor-β-D-ribofuranosyl)-5-hydroxymethyluracil

Das aus 2,88 g (10 mmol) 5'-O-Acetyl-3'-fluorthymidin in Stufe a) erhaltene Bromierungsprodukt wurde in 50 ml 1,4-Dioxan gelöst, mit 30 ml gesättigter Natriumhydrogencarbonatlösung versetzt und 1 h bei Raumtemperatur gerührt. Anschließend wurde die Reaktionslösung mit 5 × 30 ml Chloroform extrahiert. Die vereinigten Chloroformextrakte wurden über Natriumsulfat getrocknet, filtriert und im Vakuum bis zur Bildung eines sirupösen Öls eingeengt. Dieses Produkt lieferte nach dem üblichen Behandeln mit 50 ml Methanol/Ammoniak (bei 0°C gesättigt) die Titelverbindung, die aus Ethanol kristallin erhalten wurde.

Beispiel 3

Herstellung von 2',3'-Didesoxy-3'-fluor-purinribosiden durch Fluorierung von entsprechenden Xylo-Derivaten mit Dialkylaminoschwefeltrifluorid

9-(2,3-Didesoxy-3-fluor-β-D-ribofuranosyl)-2-fluoradenosin (2',3'-Didesoxy-3'-fluor-2-fluoradenosin)

10 mmol 9-(5-O-Acyl-2-desoxy-β-D-xylofuranosyl)-2-fluoradenosin wurden in 10 ml Chloroform gelöst und zu einer auf -78°C gekühlten Lösung von 11 mmol Diethylaminoschwefeltrifluorid in 30 ml Chloroform gegeben. Das Reaktionsgemisch wurde langsam auf Raumtemperatur erwärmt und in 100 ml Eiswasser gegeben. Die organische Phase wurde abgetrennt, mit Natriumhydrogencarbonatlösung und Wasser gewaschen und über Natriumsulfat getrocknet. Nach dem Vertreiben des Chloroforms im Vakuum wurde ein Produkt erhalten, dessen O-Acylgruppe in bekannter Weise abgespalten wurde. Eine säulenchromatographische Trennung an Kieselgel mit Chloroform (5% Methanol) als Elutionsmittel lieferte die Titelverbindung als Feststoff.

Beispiel 4

Transglykosidierung zur Herstellung von 2- bzw. 2,6-substituierten 2',3'-Didesoxy-3'-fluor-purinribosiden

9-(2,3-Didesoxy-3-fluor-β-D-ribofuranosyl)-2-fluoradenin

Ein Gemisch von 2,88 g (10 mmol) 1-(5-O-Acetyl-2,3-didesoxy-3-fluor-β-D-ribofuranosyl)-thymin, 4,6 g

(30 mmol) 2-Fluoradenin, 7,4 ml Bis(trimethylsilyl)-acetamid und 250 ml Acetonitril wurden 25 min am Rückfluß erhitzt. Anschließend wurden 6,5 ml (33 mmol) Trifluormethansulfonsäure-trimethylsilylester hinzugefügt, und die Reaktionslösung wurde weitere 8 h lang am Rückfluß erwärmt. Nach dem Enffernen des Lösungsmittels im Vakuum wurde der Rückstand in 100 ml Chloroform suspendiert und mit $NaHCO_3$-Lösung neutralisiert. Die organische Phase wurde im Vakuum über Natriumsulfat getrocknet und filtriert; das Chloroform wurde im Vakuum enffernt. Der Rückstand wurde an Kieselgel säulenchromatographisch getrennt, wobei mit Chloroform (5% n-Hexan, 1 l; 2,5% n-Hexan, 1,5 l; 1% n-Hexan, 1 l) eluiert wurde.

Das erhaltene α/(β-Gemisch wurde in üblicher Weise mittels Ammoniak-Methanol entacetyliert und einer säulenchromatographischen Trennung unterzogen. Als Elutionsmittel wurde Chloroform (1% Methanol) verwendet. Vom β-Anomer, der Titelverbindung, wurden 0,51 g isoliert. Weiteres Produkt verblieb im Gemisch mit dem α-Anomer.

Beispiel 5

Herstellung von 5-substituierten 2',3'-Didesoxy-3'-fluorcytidinen aus den entsprechenden Uridinen

1-(2,3-Didesoxy-3-fluor-β-D-ribofuranosyl)-5-methylcytidin (2',3'-Didesoxy-3'-fluor-5-methylcytidin)

1,5 g (5,5 mmol) 5'-O-Acetyl-3'-fluor-thymidin wurden in 25 ml Pyridin gelöst und mit 760 mg (11 mmol) Triazol und 1,96 g (8 mmol) β-Chlorphenoxyphosphorsäuredichlorid versetzt. Das Reaktionsgemisch wurde 5 h bei Raumtemperatur belassen. Anschließend wurden 30 ml Dioxan/konz. Ammoniak (Volumenverhältnis 3:1) hinzugefügt.

Die Lösung wurde dann im Vakuum eingeengt; der resultierende Rückstand in Wasser gelöst und auf eine 70-ml-Säule eines Ionenaustauscherharzes in der $H^+$-Form (DOWEX 50 W × 8, DOW Chemical Laboratories) gegeben. Die Säule wurde zunächst mit 800 ml Wasser und anschließend mit 600 ml 5%-iger Ammoniaklösung eluiert. Aus den entsprechenden Fraktionen, die UV-absorbierendes Produkt enthielten, konnten nach dem Vertreiben des Lösungsmittels und Kristallisation aus Methanol (mit HCl auf pH 2 eingestellt) 0,8 g 2',3'-Didesoxy-3'-fluor-5-methylcytosin als Hydrochlorid gewonnen werden. F. 177°C (Zers.).

Beispiel 6

Herstellung von 3'-Desoxy-3'-fluorarabinosylcytosin aus 3'-Desoxy-3'-fluorarabinosyluracil

1-(3'-Desoxy-3'-fluor-β-D-arabinosyl)-cytosin

Ein Gemisch von 2,46 g (10 mmol) 3'-Desoxy-3'-fluorarabinosyluracil, 1,52 g (22 mmol) Triazol und 4,0 g (16 mmol) p-Chlorphenoxyphosphorsäuredichlorid in 50 ml Pyridin wurde wie in Beispiel 5 behandelt.

Nach der Trennung des Reaktionsgemisches an einem Ionenaustauscher in der $H^+$-Form (DOWEX 50 W × 8) wurden aus Methanol/HCl (pH 2) 1,53 g der Titelverbindung als Hydrochlorid erhalten.

Beispiel 7

Injektionslösung

Aus 3'-FTdR und physiologischer Kochsalzlösung wird die erforderliche Menge einer 0,3-%igen Lösung hergestellt.

Beispiel 8

Tabletten und Dragées

Pulverisiertes 3'-FTdR wird mit einem oder mehreren üblichen Trägerstoffen, wie Stärke, Talk, Magnesiumstearat, Kaliumstearat, Stearinsäure, Paraffin, Cetylalkohol, Pectin, Saccharose, Gummi arabicum oder Dextrin, zu Tabletten oder Dragées verarbeitet.

**Patentansprüche**

**Patentansprüche für folgende Vertragsstaaten : BE, CH, DE, FR, GB, IT, LI, LU, NL, SE**

**1.** 3'-fluorierte Pyrimidin- bzw. Purinnucleoside der allgemeinen Formel I.

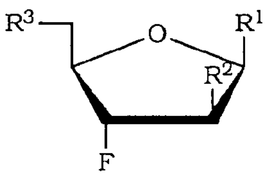

(I)

worin bedeuten:
$R^1$ einen N-1-glykosidisch gebundenen Rest, der von Thymin, Uracil, einem 5-substituierten Uracil, Cytosin oder einem 5-substituierten Cytosinderivat abgeleitet ist,
oder einen N-9-glykosidisch gebundenen Rest, der von 2-Fluoradenin, 2,6-Diaminopurin, 2-Aminopurin, 6-Thioguanin oder 7-Deazaadenin abgeleitet ist,
$R^2$ H oder OH und
$R^3$ OH, O-Acetyl. O-Palmitoyl, Triphosphat als freie Säure oder in Form entsprechender Alkali-, Ammonium-bzw. Alkylammoniumsalze oder eine Prekursor-Gruppe für die Hydroxygruppe,
wobei diejenigen Verbindungen der Fonmel I ausgenommen sind, in der bedeuten:
$R^1$ einen N-1-glykosidisch gebundenen Rest, der von Thymin, Uracil, einem 5-substituierten Uracil, Cytosin oder einem 5-substituierten Cytosinderivat abgeleitet ist,
$R^2$ H und
$R^3$ OH, O-Acetyl, O-Palmitoyl, Triphosphat als freie Säure oder in Form entsprechender Alkali-, Ammonium- bzw. Alkylammoniumsalze oder eine Prekursor-Gruppe für die Hydroxygruppe.

2. 2',3'-Didesoxy-3'-fluor-5-bromuridin.

3. 2',3'-Didesoxy-3'-fluor-5-ethyluridin.

4. 2',3'-Didesoxy-3'-fluor-2-fluoradenosin.

5. 2',3'-Didesoxy-3'-fluor-6-thioguanosin.

6. 2',3'-Didesoxy-3'-fluor-2,6-diaminopurinribosid.

7. 2',3'-Didesoxy-3'-fluor-2-aminopurinribosid.

8. Verfahren zur Herstellung der 3'-fluorierten Pyrimidinnucleoside der Formel 1 nach einem der Ansprüche 1 bis 7, **dadurch gekennzeichnet,** daß man - zur Herstellung von 5-Brom- oder 5-Ethyl- 2',3'-didesoxy-3'-fluoruridinen entsprechende, 5-substituierte 2',3'-Anhydro-2'-desoxyuridine mit HF umsetzt;
   - zur Herstellung von ggf. 5-substituierten 3'-Desoxy-3'-fluorarabinosylcytosinen die entsprechenden, ggf. 5-substituierten Cytosinderivate in die Triazolylderivate überführt und daraus die Zielverbindungen herstellt und
   - zur Herstellung von 2- bzw. 2,6-substituierten 2',3'-Didesoxy-3'-fluorpurinribosiden die entsprechenden Xyloribofuranosylderivate, die in 5'-Position acyliert sind, mit Dialkylaminoschwefeltrifluorid umsetzt oder den fluorierten Zuckerrest aus 2',3'-Didesoxy-3'-fluorthymidin (3'-FTdR) durch Transglykosidierung auf das entsprechend modifizierte Purinderivat überträgt.

9. Pharmazeutische Mittel, **gekennzeichnet durch** ein oder mehrere 3'-fluorierte Pyrimidin- bzw. Purinnucleoside der Formel I nach einem der Ansprüche 1 bis 7 als Wirkstoffe neben pharmazeutisch geeigneten Hilfs- und/oder Trägerstoffen.

10. Verwendung
   - der 3'-fluorierten Pyrimidin- bzw. Purinnucleoside der Formel I nach einem der Ansprüche 1 bis 7, wobei auch diejenigen Verbindungen der Formel I inbegriffen sind, in der $R^1$ einen N-1-glykosidisch gebundenen, von Uracil, einem 5-substituierten Uracil (mit Ausnahme von Thymin) oder einem 5-substituierten Cytosinderivat abgeleiteten Rest,
     $R^2$ H und
     $R^3$ OH
     bedeuten,
   - und/oder mindestens einer unter 3'-Desoxy-3'-fluorarabinosylcytosin und 3'-Desoxy-3'-fluorarabinosyladenin ausgewählten Verbindung zur Herstellung pharmazeutischer Mittel zur Prophylaxe

und zur Bekämpfung der erworbenen Immunschwäche AIDS, insbesondere in der Humanmedizin.

**11.** Pharmazeutische Mittel nach Anspruch 9, **dadurch gekennzeichnet,** daß sie für die Verabreichung durch Injektion, Infusion oder für die orale Verabreichung formuliert sind.

**12.** Pharmazeutische Mittel nach Anspruch 9 oder 11, **dadurch gekennzeichnet,** daß sie in Form von Dragées, Tabletten, Kapseln, in Pulverform, als Granulate oder in Form von Zäpfchen konfektioniert sind.

**13.** Pharmazeutische Mittel nach einem der Ansprüche 9, 11 oder 12, **dadurch gekennzeichnet,** daß sie in Einheiten konfektioniert sind, die eine oder mehrere Einheitsdosen des bzw. der Wirkstoffe enthalten.

**14.** Verfahren zur Herstellung der pharmazeutischen Mittel nach einem der Ansprüche 9 und 11 bis 13, **dadurch gekennzeichnet**, daß man den bzw. die Wirkstoffe zusammen mit üblichen Hilfs- und/oder Trägerstoffen in eine für die betreffende Darreichungsart geeignete pharmazeutische Fonm bringt.

**15.** Verwendung der Verbindungen gemäß Anspruch 10 zur Herstellung pharmazeutischer Mittel für die Verabreichung durch Injektion, Infusion oder für orale Verabreichung.

**16.** Verwendung der Verbindungen gemäß Anspruch 10 oder 15 zur Herstellung pharmazeutischer Mittel in Form von Dragées, Tabletten, Kapseln, Granulaten oder Zäpfchen oder in Pulverform.

**17.** Verwendung der Verbindungen gemäß einem der Ansprüche 10, 15 und 16, zur Herstellung pharmazeutischer Mittel, die in Einheiten konfektioniert sind, die eine oder mehrere Einheitsdosen des bzw. der Wirkstoffe enthalten.

**Patentanspruch für folgende Vertragsstaat : ES**

**1.** Verfahren zur Herstellung von 3'-fluorierten Pyrimidin- bzw. Purinnucleosiden der allgemeinen Formel (I)

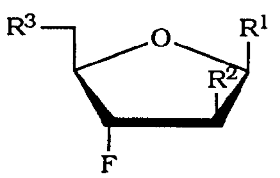

(I)

worin bedeuten:

$R^1$ einen N-1-glykosidisch gebundenen Rest, der von Thymin, Uracil, einem 5-substituierten Uracil, Cytosin oder einem 5-substituierten Cytosinderivat abgeleitet ist,

oder einen N-9-glykosidisch gebundenen Rest, der von 2-Fluoradenin, 2,6-Diaminopurin, 2-Aminopurin, 6-Thioguanin oder 7-Deazaadenin abgeleitet ist,

$R^2$ H oder OH und

$R^3$ OH, O-Acetyl, O-Palmitoyl, Triphosphat als freie Säure oder in Form entsprechender Alkali-, Ammonium-bzw. Alkylammoniumsalze oder eine Prekursor-Gruppe für die Hydroxygruppe,

wobei diejenigen Verbindungen der Formel I ausgenommen sind, in der bedeuten:

$R^1$ einen N-1-glykosidisch gebundenen Rest, der von Thymin, Uracil, einem 5-substituierten Uracil (mit Ausnahme von Thymin), Cytosin oder einem 5-substituierten Cytosinderivat abgeleitet ist,

$R^2$ H, und

$R^3$ OH,

**dadurch gekennzeichnet** daß man

- zur Herstellung von 5-Brom- oder 5-Ethyl-2',3'-didesoxy-3'-fluoruridinen entsprechende, 5-substituierte 2',3'-Anhydro-2'-desoxyuridine mit HF umsetzt;
- zur Herstellung von ggf. 5-substituierten 3'-Desoxy-3'- fluorarabinosylcytosinen die entsprechenden, ggf. 5-substituierten Cytosinderivate in die Triazolylderivate überführt und daraus die Zielverbindungen herstellt und
- zur Herstellung von 2- bzw. 2,6-substituierten 2',3'-Didesoxy-3'-fluorpurinribosiden die entsprechenden Xyloribofuranosylderivate, die in 5'-Position acyliert sind, mit Dialkylaminoschwefeltrifluorid umsetzt oder

den fluorierten Zuckerrest aus 2',3'-Didesoxy-3'-fluorthymidin (3'-FTdR) durch Transglykosidierung auf das entsprechend modifizierte Purinderivat überträgt.

**Patentansprüche für folgende Vertragsstaat : GR**

1. Verfahren zur Herstellung von 3'-fluorierten Pyrimidin- bzw. Purinnucleosiden der allgemeinen Formel (I)

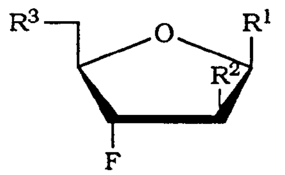

(I)

worin bedeuten:
$R^1$ einen N-1-glykosidisch gebundenen Rest, der von Thymin, Uracil, einem 5-substituierten Uracil, Cytosin oder einem 5-substituierten Cytosinderivat abgeleitet ist,
oder einen N-9-glykosidisch gebundenen Rest, der von 2-Fluoradenin, 2,6-Diaminopurin, 2-Aminopurin, 6-Thioguanin oder 7-Deazaadenin abgeleitet ist,
$R^2$ H oder OH und
$R^3$ OH, O-Acetyl, O-Palmitoyl, Triphosphat als freie Säure oder in Form entsprechender Alkali-, Ammonium-bzw. Alkylammoniumsalze oder eine Prekursor-Gruppe für die Hydroxygruppe,
wobei diejenigen Verbindungen der Formel I ausgenommen sind, in der bedeuten:
$R^1$ einen N-1-glykosidisch gebundenen Rest, der von Thymin, Uracil, einem 5-substituierten Uracil (mit Ausnahme von Thymin), Cytosin oder einem 5-substituierten Cytosinderivat abgeleitet ist,
$R^2$ H, und
$R^3$ OH,
**dadurch gekennzeichnet,** daß man
- zur Herstellung von 5-Brom- oder 5-Ethyl-2',3'-didesoxy-3'-fluoruridinen entsprechende, 5-substituierte 2',3'-Anhydro-2'-desoxyuridine mit HF umsetzt;
- zur Herstellung von ggf. 5-substituierten 3'-Desoxy-3'- fluorarabinosylcytosinen die entsprechenden, ggf. 5-substituierten Cytosinderivate in die Triazolylderivate überführt und daraus die Zielverbindungen herstellt und
- zur Herstellung von 2- bzw. 2,6-substituierten 2',3'-Didesoxy-3'-fluorpurinribosiden die entsprechenden Xyloribofuranosylderivate, die in 5'-Position acyliert sind, mit Dialkylaminoschwefeltrifluorid umsetzt oder
  den fluorierten Zuckerrest aus 2',3'-Didesoxy-3'-fluorthymidin (3'-FTdR) durch Transglykosidierung auf das entsprechend modifizierte Purinderivat überträgt.

2. Verwendung der nach dem Verfahren gemäß Anspruch 1 erhaltenen 3'-fluorierten Pyrimidin- bzw. Purinnucleoside der Formel (I), wobei auch diejenigen Verbindungen der Formel I inbegriffen sind, in der $R^1$ einen N-1-glykosidisch gebundenen, von Uracil, einem 5-substituierten Uracil (mit Ausnahme von Thymin), Cytasin oder einem 5-substituierten Cytosinderivat abgeleiteten Rest,
$R^2$ H und
$R^3$ OH
bedeuten,
- und/oder mindestens einer unter 2',3'-Didesoxy-3'-fluorthymidin (3'-FTdR), 2',3'-Didesoxy-3'-fluorcytidin, 3'-Desoxy-3'-fluorarabinosylcytosin und 3'-Desoxy-3'-fluor- arabinosyladenin ausgewählten Verbindung zur Herstellung pharmazeutischer Mittel zur Prophylaxe und zur Bekämpfung der erworbenen Immunschwäche AIDS, insbesondere in der Humanmedizin.

3. Verwendung nach Anspruch 2 zur Herstellung pharmazeutischer Mittel für die Verabreichung durch Injektion, Infusion oder für orale Verabreichung.

4. Verwendung gemäß Anspruch 2 oder 3 zur Herstellung pharmazeutischer Mittel in Form von Dragees, Tabletten, Kapseln, Granulaten oder Zäpfchen oder in Pulverform.

5. Verwendung gemäß den Ansprüchen 2 bis 4 zur Herstellung pharmazeutischer Mittel, die in Einheiten

konfektioniert sind, die eine oder mehrere Einheitsdosen des bzw. der Wirkstoffe enthalten.

## Claims

**Claims for the following Contracting States : BE, CH, DE, FR, GB, IT, LI, LU, NL, SE**

1. 3'-Fluorinated pyrimidine or purine nucleosides of general formula I:

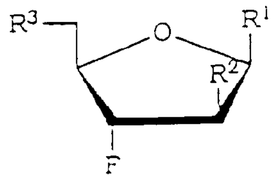

(I)

in which:
$R^1$ is an N-1-glycosidically linked radical derived from thymine, uracil, a 5-substituted uracil, cytosine or a 5-substituted cytosine derivative,
or an N-9-glycosidically linked radical derived from 2-fluoroadenine, 2,6-diaminopurine, 2-aminopurine, 6-thioguanine or 7-deazaadenine,
$R^2$ is H or OH and
$R^3$ is OH, O-acetyl, O-palmitoyl, triphosphate as the free acid or in the form of corresponding alkali metal, ammonium or alkylammonium salts, or a precursor group for the hydroxy group,
with the exception of those compounds of formula I in which:
$R^1$ is an N-1-glycosidically linked radical derived from thymine, uracil, a 5-substituted uracil, cytosine or a 5-substituted cytosine derivative,
$R^2$ is H and
$R^3$ is OH, O-acetyl, O-palmitoyl, triphosphate as the free acid or in the form of corresponding alkali metal, ammonium or alkylammonium salts, or a precursor group for the hydroxy group.

2. 2',3'-Dideoxy-3'-fluoro-5-bromouridine.

3. 2',3'-Dideoxy-3'-fluoro-5-ethyluridine.

4. 2',3'-Dideoxy-3'-fluoro-2-fluoroadenosine.

5. 2',3'-Dideoxy-3'-fluoro-6-thioguanosine.

6. 2',3'-Dideoxy-3'-fluoro-2,6-diaminopurine riboside.

7. 2',3'-Dideoxy-3'-fluoro-2-aminopurine riboside.

8. A process for the preparation of the 3'-fluorinated pyrimidine nucleosides of formula I according to one of Claims 1 to 7, **characterized in that**
   - 5-bromo- or 5-ethyl-2',3'-dideoxy-3'-fluorouridines are prepared by reacting the corresponding 5-substituted 2',3'-anhydro-2'-deoxyuridines with HF;
   - optionally 5-substituted 3'-deoxy-3'-fluoroarabinosylcytosines are prepared by converting the corresponding optionally 5-substituted cytosine derivatives to the triazolyl derivatives and preparing the target compounds therefrom; and
   - 2- or 2,6-substituted 2',3'-dideoxy-3'-fluoropurine ribosides are prepared by reacting the corresponding xyloribofuranosyl derivatives, acylated in the 5'- position, with dialkylaminosulphur trifluoride, or transferring the fluorinated sugar residue from 2',3'- dideoxy-3'-fluorothymidine (3'-FTdR) to the correspondingly modified purine derivative by transglycosidation.

9. Pharmaceutical compositions, **characterized in that** they contain one or more 3'-fluorinated pyrimidine or purine nucleosides of formula I according to one of Claims 1 to 7 as active ingredients, together with pharmaceutically acceptable adjuncts and/or excipients.

**10.** Use of

- the 3'-fluorinated pyrimidine or purine nucleosides of formula I according to one of Claims 1 to 7, including those compounds of formula I in which $R^1$ is an N-1- glycosidically linked radical derived from uracil, a 5-substituted uracil (except for thymine) or a 5-substituted cytosine derivative,
  $R^2$ is H and
  $R^3$ is OH, and/or
- at least one compound selected from 3'-deoxy-3'-fluoroarabinosylcytosine and 3'-deoxy-3'-fluoroarabinosyladenine,
  for the preparation of pharmaceutical compositions for the prophylaxis and control of the acquired immune deficiency AIDS, especially in human medicine.

**11.** Pharmaceutical compositions according to Claim 9, **characterized in that** they are formulated for administration by injection or infusion or for oral administration.

**12.** Pharmaceutical compositions according to Claim 9 or 11, **characterized in that** they are manufactured in the form of coated tablets, tablets or capsules, in powder form, as granules or in the form of suppositories.

**13.** Pharmaceutical compositions according to one of Claims 9, 11 or 12, **characterized in that** they are manufactured in units containing one or more unit doses of the active ingredient or ingredients.

**14.** A process for the preparation of the pharmaceutical compositions according to one of Claims 9 and 11 to 13, **characterized in that** the active ingredient or ingredients, together with conventional adjuncts and/or excipients, are converted to a pharmaceutical form suitable for the relevant mode of administration.

**15.** Use of the compounds according to Claim 10 for the preparation of pharmaceutical compositions for administration by injection or infusion or for oral administration.

**16.** Use of the compounds according to Claim 10 or 15 for the preparation of pharmaceutical compositions in the form of coated tablets, tablets, capsules, granules or suppositories or in powder form.

**17.** Use of the compounds according to one of Claims 10, 15 and 16 for the preparation of pharmaceutical compositions which are manufactured in units containing one or more unit doses of the active ingredient or ingredients.

**Claim for the following Contracting State : ES**

**1.** A process for the preparation of 3'-fluorinated pyrimidine or purine nucleosides of general formula (I):

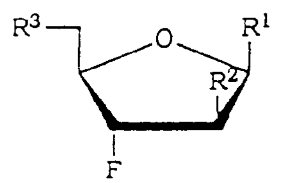

(I)

in which:
$R^1$ is an N-1-glycosidically linked radical derived from thymine, uracil, a 5-substituted uracil, cytosine or a 5-substituted cytosine derivative,
or an N-9-glycosidically linked radical derived from 2-fluoroadenine, 2,6-diaminopurine, 2-aminopurine, 6-thioguanine or 7-deazaadenine,
$R^2$ is H or OH and
$R^3$ is OH, O-acetyl, O-palmitoyl, triphosphate as the free acid or in the form of corresponding alkali metal, ammonium or alkylammonium salts, or a precursor group for the hydroxy group,
with the exception of those compounds of formula I in which:
$R^1$ is an N-1-glycosidically linked radical derived from thymine, uracil, a 5-substituted uracil (except for thymine), cytosine or a 5-substituted cytosine derivative,
$R^2$ is H and

$R^3$ is OH,

**characterized in that**

- 5-bromo- or 5-ethyl-2',3'-dideoxy-3'-fluorouridines are prepared by reacting the corresponding 5-substituted 2',3'-anhydro-2'-deoxyuridines with HF;
- optionally 5-substituted 3'-deoxy-3'-fluoroarabinosylcytosines are prepared by converting the corresponding optionally 5-substituted cytosine derivatives to the triazolyl derivatives and preparing the target compounds therefrom; and
- 2- or 2,6-substituted 2',3'-dideoxy-3'-fluoropurine ribosides are prepared by reacting the corresponding xyloribofuranosyl derivatives, acylated in the 5'- position, with dialkylaminosulphur trifluoride, or transferring the fluorinated sugar residue from 2',3'-dideoxy-3'-fluorothymidine (3'-FTdR) to the correspondingly modified purine derivative by transglycosidation.

**Claims for the following Contracting State : GR**

**1.** A process for the preparation of 3'-fluorinated pyrimidine or purine nucleosides of general formula (I):

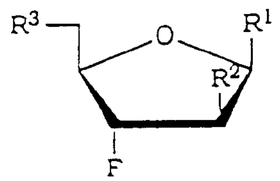

(I)

in which:

$R^1$ is an N-1-glycosidically linked radical derived from thymine, uracil, a 5-substituted uracil, cytosine or a 5-substituted cytosine derivative,

or an N-9-glycosidically linked radical derived from 2-fluoroadenine, 2,6-diaminopurine, 2-aminopurine, 6-thioguanine or 7-deazaadenine,

$R^2$ is H or OH and

$R^3$ is OH, O-acetyl, O-palmitoyl, triphosphate as the free acid or in the form of corresponding alkali metal, ammonium or alkylammonium salts, or a precursor group for the hydroxy group,

with the exception of those compounds of formula I in which:

$R^1$ is an N-1-glycosidically linked radical derived from thymine, uracil, a 5-substituted uracil (except for thymine), cytosine or a 5-substituted cytosine derivative,

$R^2$ is H and

$R^3$ is OH,

**characterized in that**

- 5-bromo- or 5-ethyl-2',3'-dideoxy-3'-fluorouridines are prepared by reacting the corresponding 5-substituted 2',3'-anhydro-2'-deoxyuridines with HF;
- optionally 5-substituted 3'-deoxy-3'-fluoroarabinosylcytosines are prepared by converting the corresponding optionally 5-substituted cytosine derivatives to the triazolyl derivatives and preparing the target compounds therefrom; and
- 2- or 2,6-substituted 2',3'-dideoxy-3'-fluoropurine ribosides are prepared by reacting the corresponding xyloribofuranosyl derivatives, acylated in the 5'- position, with dialkylaminosulphur trifluoride, or transferring the fluorinated sugar residue from 2',3'-dideoxy-3'-fluorothymidine (3'-FTdR) to the correspondingly modified purine derivative by transglycosidation.

**2.** Use of

- the 3'-fluorinated pyrimidine or purine nucleosides of formula (I) obtained by the process according to Claim 1, including those compounds of formula I in which $R^1$ is an N-1-glycosidically linked radical derived from uracil, a 5-substituted uracil (except for thymine), cytosine or a 5-substituted cytosine derivative,
  $R^2$ is H and
  $R^3$ is OH, and/or
- at least one compound selected from 2',3'- dideoxy- 3'-fluorothymidine (3'-FTdR), 2',3'- dideoxy-3'-fluorocytidine, 3'-deoxy-3'-fluoroarabinosylcytosine and 3'-deoxy-3'-fluoroarabinosyladenine,
  for the preparation of pharmaceutical compositions for the prophylaxis and control of the acquired immune deficiency AIDS, especially in human medicine.

3. Use according to Claim 2 for the preparation of pharmaceutical compositions for administration by injection or infusion or for oral administration.

4. Use according to Claim 2 or 3 for the preparation of pharmaceutical compositions in the form of coated tablets, tablets, capsules, granules or suppositories or in powder form.

5. Use according to Claims 2 to 4 for the preparation of pharmaceutical compositions which are manufactured in units containing one or more unit doses of the active ingredient or ingredients.

**Revendications**

**Revendications pour les Etats contractants suivants : BE, CH, DE, FR, GB, IT, LI, LU, NL, SE**

1. Nucléosides de la pyrimidine ou de la purine 3'-fluorés suivant la formule générale (I) :

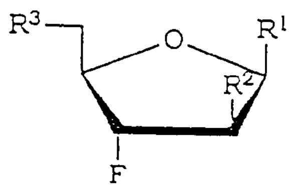

(I)

dans laquelle :

$R^1$ représente un radical à liaison N-1-glycosidique qui provient de la thymine, de l'uracile, d'un uracile 5-substitué, de la cytosine ou d'un dérivé 5-substitué de la cytosine,
ou un radical à liaison N-9-glycosidique qui provient de la 2-fluoroadénine, de la 2,6-diaminopurine, de la 2-aminopurine, de la 6-thioguanine ou de la 7-déazadénine,

$R^2$ représente H ou OH, et

$R^3$ représente OH, O-acétyle, O-palmitoyle, un triphosphate sous forme d'acide libre ou sous forme des sels correspondants de métaux alcalins, d'ammonium ou d'alcoylammonium ou un groupe précurseur pour le groupe hydroxy,

dans laquelle sont exclus les composés de la formule (I) dans laquelle :

$R_1$ représente un radical à liaison N-1-glycosidique qui provient de la thymine, de l'uracile, d'un uracile-5-substitué, de la cytosine ou d'un dérivé 5-substitué de la cytosine,

$R^2$ représente H, et

$R^3$ représente OH, O-acétyle, O-palmitoyle, un triphosphate sous forme d'acide libre ou sous forme des sels correspondants de métaux alcalins, d'ammonium ou d'alcoylammonium ou un groupe précurseur pour le groupe hydroxy.

2. 2',3'-didésoxy-3'-fluoro-5-bromuridine.

3. 2',3 '-didésoxy-3'-fluoro-5-éthyluridine.

4. 2',3'-didésoxy-3'-fluoro-2-fluoradénosine.

5. 2',3'-didésoxy-3'-fluoro-6-thioguanosine.

6. 2',3'-didésoxy-3'-fluoro-2,6-diaminopurineriboside

7. 2',3'-didésoxy-3'-fluoro-2-aminopurineriboside.

8. Procédé pour la préparation des nucléosides de la pyrimidine 3'-fluorée suivant la formule (I), conformément à l'une des revendications 1 à 7, caractérisé en ce que :
   - pour la préparation des 5-bromo- ou 5-éthyl-2',3'-didésoxy-3'-fluoruridines, on fait réagir avec HF les 2',3'-anhydro-2'-désoxyuridines 5-substituées correspondantes,
   - pour la préparation des 3'-désoxy-3'-fluorarabinosylcytosines éventuellement 5-substituées, on transforme les dérivés cytosine éventuellement 5-substitués correspondants en dérivés triazolyle et,

à partir de ceux-ci, on prépare les composés voulus;

- pour la préparation des 2',3'-didésoxy-3'-fluoropurineribosides 2- ou 2,6-substitués, on fait réagir avec un trifluorure de dialcoylaminosoufre les dérivés correspondants xyloribofuranosyle qui sont acylés en position 5', ou on transfère le reste sucre fluoré de la 2',3'-didésoxy-3'-fluorothymidine (3'-FTdR) par transglycosidation sur le dérivé purine modifié correspondant.

**9.** Produit pharmaceutique, caractérisé par un ou plusieurs nucléosides de pyrimidine ou de purine 3'-fluorés suivant la formule (I) et conformément à l'une des revendications 1 à 7 utilisés comme substances actives en combinaison avec des substances auxiliaires et/ou de supports pharmaceutiquement appropriés.

**10.** Utilisation

- des nucléosides de pyrimidine ou de purine 3'-fluorés suivant la formule (I), conformément à l'une des revendications 1 à 7, dans laquelle sont également compris des composés de la formule (I) dans lesquels :

  $R^1$ représente un radical à liaison N-1-glycosidique provenant de l'uracile, d'un uracile-5-substitué (à l'exception de la thymine) ou d'un dérivé 5-substitué de la cytosine,

  $R^2$ représente H

  et

  $R^3$ représente OH;

- et/ou au moins un composé choisi parmi la 3'-désoxy-3'-fluorarabinosylcytosine et la 3'-désoxy-3'-fluorarabinosyladénine pour obtenir des produits pharmaceutiques en vue du traitement prophylactique et thérapeutique du syndrome d'immunodéficience acquise SIDA et, en particulier en médecine humaine.

**11.** Produit pharmaceutique selon la revendication 9, caractérisé en ce que celui-ci est formulé en vue de l'administration par injection, par perfusion ou par voie orale.

**12.** Produit pharmaceutique selon la revendication 9 ou 11, caractérisé en ce qu'il est préparé sous forme de dragées, de tablettes, de capsules, de poudres, de granulés ou de suppositoires.

**13.** Produit pharmaceutique selon l'une des revendications 9, 11 ou 12, caractérisé en ce qu'il est préparé sous forme d'unités contenant une ou plusieurs doses unitaires de l'agent ou des agents actif(s).

**14.** Procédé pour la préparation de produit pharmaceutique selon l'une des revendications 9 et 11 à 13, caractérisé en ce que l'on met la ou les substance(s) active(s) combinée(s) aux substances auxiliaires et/ou de support usuelles sous une forme pharmaceutique convenant pour le mode d'administration envisagé.

**15.** Utilisation des composés selon la revendication 10 pour la préparation de produit pharmaceutique, en vue de leur administration par injection, par perfusion ou par voie orale.

**16.** Utilisation des composés selon la revendication 10 ou 15 pour la préparation de produit pharmaceutique sous forme de dragées, de tablettes, de capsules, de granulés ou de suppositoires, ou encore sous forme de poudres.

**17.** Utilisation des composés selon l'une des revendications 10, 15 et 16, pour la préparation de produit pharmaceutique qui est préparé sous forme d'unités contenant une ou plusieurs doses unitaires de la ou des substances(s) active(s).

**Revendication pour l'Etat contractant suivant : ES**

**1.** Procédé de préparation de nucléosides de la pyrimidine ou de la purine 3'-fluorés suivant la formule générale (I) :

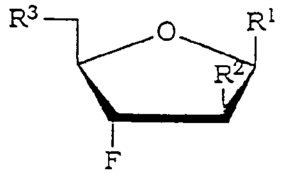

(I)

dans laquelle :

$R^1$ représente un radical à liaison N-1-glycosidique qui provient de la thymine, de l'uracile, d'un uracile 5-substitué, de la cytosine ou d'un dérivé 5-substitué de la cytosine,

ou un radical à liaison N-9-glycosidique qui provient de la 2-fluoroadénine, de la 2,6-diaminopurine, de la 2-aminopurine, de la 6-thioguanine ou de la 7-déazadénine,

$R^2$ représente H ou OH, et

$R^3$ représente OH, O-acétyle, O-palmitoyle, un triphosphate sous forme d'acide libre ou sous forme des sels correspondants de métaux alcalins, d'ammonium ou d'alcoylammonium ou un groupe précurseur pour le groupe hydroxy,

dans laquelle sont exclus les composés de la formule (I) dans laquelle :

$R_1$ représente un radical à liaison N-1-glycosidique qui provient de la thymine, de l'uracile, d'un uracile-5-substitué (à l'exception de la thymine), de la cytosine ou d'un dérivé 5-substitué de la cytosine,

$R^2$ représente H, et

$R^3$ représente OH, caractérisé en ce que :

- pour la préparation des 5-bromo- ou 5-éthyl-2',3'-didésoxy-3'-fluoruridines, on fait réagir avec HF les 2',3'-anhydro-2'-désoxyuridines 5-substituées correspondantes,
- pour la préparation des 3'-désoxy-3'-fluorarabinosylcytosines éventuellement 5-substituées, on transforme les dérivés cytosine éventuellement 5-substitués correspondants en dérivés triazolyle et, à partir de ceux-ci, on prépare les composés voulus;
- pour la préparation des 2',3'-didésoxy-3'-fluoropurineribosides 2- ou 2,6-substitués, on fait réagir avec un trifluorure de dialcoylaminosoufre les dérivés correspondants xyloribofuranosyle qui sont acylés en position 5', ou on transfère le reste sucre fluoré de la 2',3'-didésoxy-3'-fluorothymidine (3'-FTdR) par transglycosidation sur le dérivé purine modifié correspondant.

**Revendications pour l'Etat contractant suivant : GR**

1. Procédé de préparation de nucléosides de la pyrimidine ou de la purine 3'-fluorés suivant la formule générale (I) :

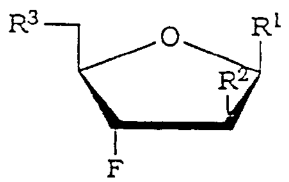

(I)

dans laquelle :

$R^1$ représente un radical à liaison N-1-glycosidique qui provient de la thymine, de l'uracile, d'un uracile 5-substitué, de la cytosine ou d'un dérivé 5-substitué de la cytosine,

ou un radical à liaison N-9-glycosidique qui provient de la 2-fluoroadénine, de la 2,6-diaminopurine, de la 2-aminopurine, de la 6-thioguanine ou de la 7-déazadénine,

$R^2$ représente H ou OH, et

$R^3$ représente OH, O-acétyle, O-palmitoyle, un triphosphate sous forme d'acide libre ou sous forme des sels correspondants de métaux alcalins, d'ammonium ou d'alcoylammonium ou un groupe précurseur pour le groupe hydroxy,

dans laquelle sont exclus les composés de la formule (I) dans laquelle :

$R_1$ représente un radical à liaison N-1-glycosidique qui provient de la thymine, de l'uracile, d'un uracile-5-substitué (à l'exception de la thymine), de la cytosine ou d'un dérivé 5-substitué de la cytosine,

$R^2$ représente H, et

$R^3$ représente OH, caractérisé en ce que :

- pour la préparation des 5-bromo- ou 5-éthyl-2',3'-didésoxy-3'-fluoruridines, on fait réagir avec HF les 2',3'-anhydro-2'-désoxyuridines 5-substituées correspondantes,
- pour la préparation des 3'-désoxy-3'-fluorarabinosylcytosines éventuellement 5-substituées, on transforme les dérivés cytosine éventuellement 5-substitués correspondants en dérivés triazolyle et, à partir de ceux-ci, on prépare les composés voulus;
- pour la préparation des 2',3'-didésoxy-3'-fluoropurineribosides 2- ou 2,6-substitués, on fait réagir avec un trifluorure de dialcoylaminosoufre les dérivés correspondants xyloribofuranosyle qui sont acylés en position 5', ou on transfère le reste sucre fluoré de la 2',3'-didésoxy-3'-fluorothymidine (3'-FTdR) par transglycosidation sur le dérivé purine modifié correspondant.

**2.** Utilisation des nucléosides de pyrimidine ou de purine 3-fluorés de formule (I), obtenus par le procédé suivant la revendication 1, dans laquelle sont également compris des composés de la formule (I) dans lesquels :

$R^1$ représente un radical à liaison N-1-glycosidique provenant de l'uracile, d'un uracile-5-substitué (à l'exception de la thymine), de la cytosine ou d'un dérivé 5-substitué de la cytosine,

$R^2$ représente H

et

$R^3$ représente OH

- et/ou au moins un composé choisi parmi la 2',3'-didésoxy-3'-fluorothymidine (3'-FTdR), la 2',3'-didésoxy-3'-fluorocytidine, la 3'-désoxy-3'-fluorarabinosylcytosine et la 3'-désoxy-3'-fluorarabinosyladénine pour obtenir des produits pharmaceutiques en vue du traitement prophylactique et thérapeutique du syndrome d'immunodéficience acquise SIDA et, en particulier en médecine humaine.

**3.** Utilisation suivant la revendication 2, pour obtenir des produits pharmaceutiques en vue de l'administration par injection, par perfusion ou par voie orale.

**4.** Utilisation suivant la revendication 2 ou 3, pour la préparation de produit pharmaceutique sous forme de dragées, de tablettes, de capsules, de granulés ou de suppositoires, ou encore sous forme de poudres.

**5.** Utilisation suivant les revendications 2 à 4, pour la préparation de produit pharmaceutique qui est préparé sous forme d'unités contenant une ou plusieurs doses unitaires de la ou des substances(s) active(s).

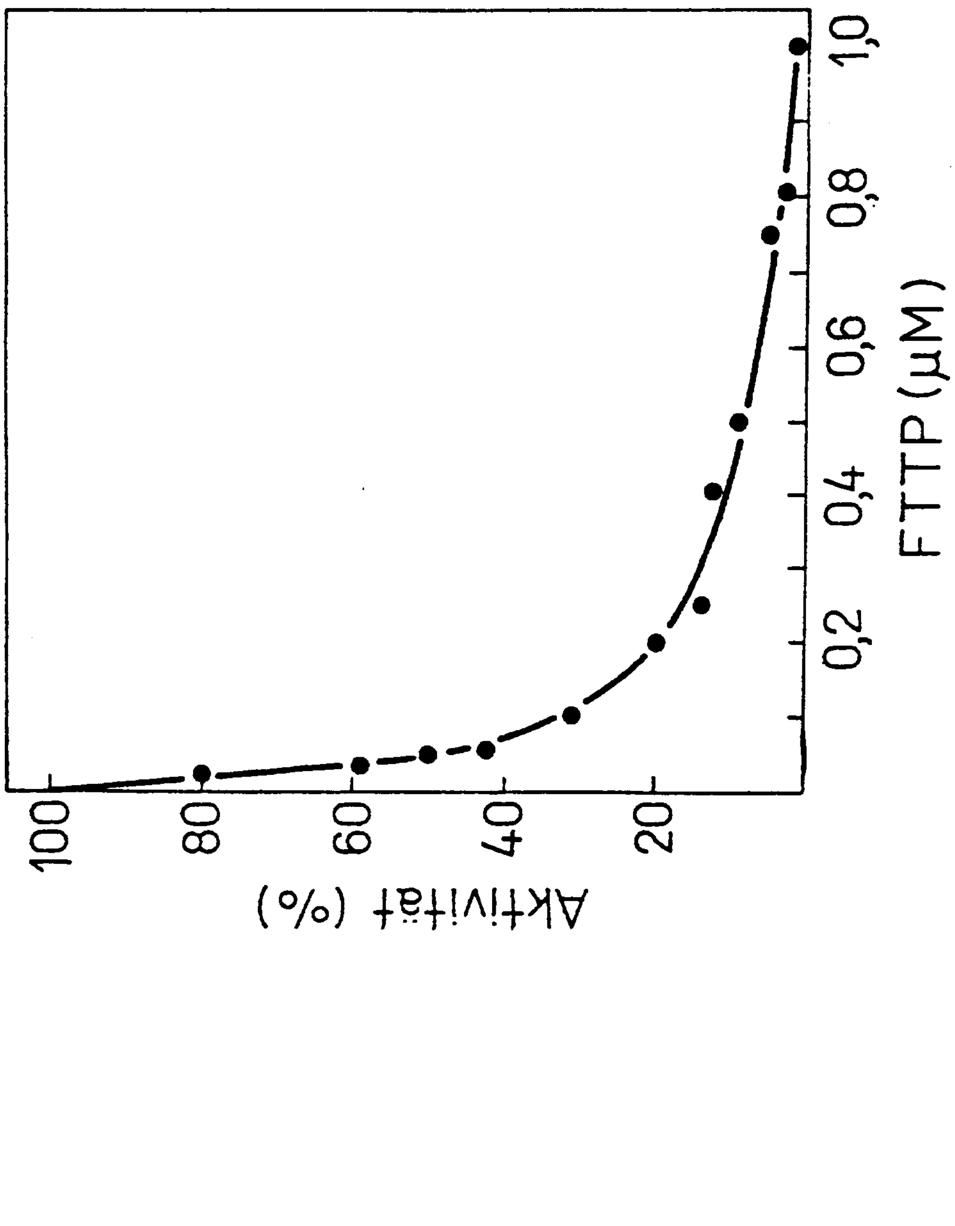


Fig. 1

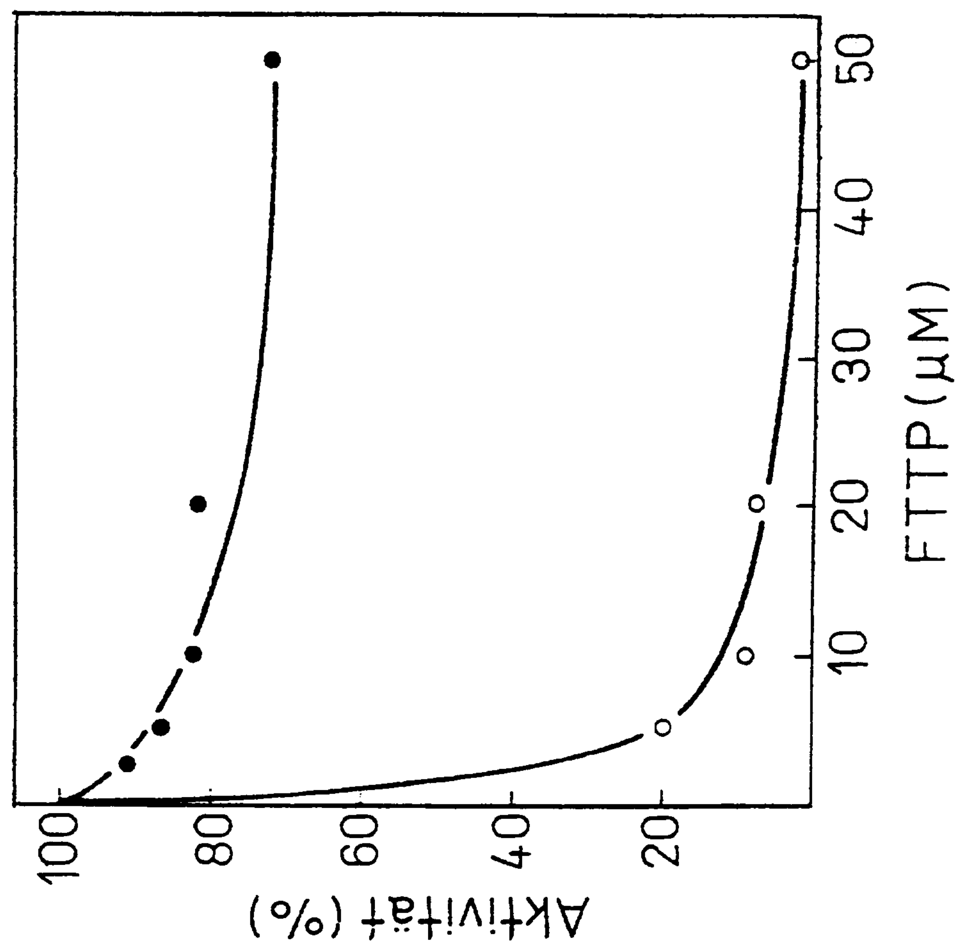


Fig. 2